# EUROPEAN PATENT APPLICATION

(11) **EP 4 389 010 A1**
(43) Date of publication of application: **26.06.2024**
(21) Application number: 22215897.4
(22) Date of filing: 22.12.2022
(51) Int. Cl.: A61B 6/03, A61B 6/00

(54) **LOCALISING POTENTIAL FAILURE EVENTS WITHIN A MEDICAL IMAGING SYSTEM**

(71) Applicant: Koninklijke Philips N.V., 5656 AG Eindhoven (NL)
(72) Inventor: AGAFONOV, Aleksei, Eindhoven (NL); VERDIESEN, Sander Jean, Eindhoven (NL); IMMINK, Albert Hendrik Jan, 5656AG Eindhoven (NL); RONDA, Cornelis Reinder, Eindhoven (NL); BASTIAANSEN, Thomas, Eindhoven (NL); WAESCHE, Martin, Eindhoven (NL); MILLEN, David, Eindhoven (NL); VOGTMEIER, Gereon, Eindhoven (NL); RIBBING, Carolina Maria, Eindhoven (NL); WLOTZKA, Oliver, Eindhoven (NL); WIECZOREK, Norbert, Eindhoven (NL); SCHOUTEN, Wim, Eindhoven (NL); WEISS, Steffen, Eindhoven (NL); FORTHMANN, Peter, Eindhoven (NL)
(74) Representative: Philips Intellectual Property & Standards

(57) **Abstract**

The present invention generally relates to medical imaging. In order to identify a location of a defect in a medical imaging system, a defect localisation system (10) for determining a location of a potential failure event within a medical imaging system. The defect localisation system comprises one or more sensors (12) and at least one processing unit (14). The one or more sensors comprise at least one of an acoustic sensor (12a), a vibration sensor (12b), and a radio-frequency sensor. The at least one processing unit is configured to receive a signal generated by the one or more sensors, and to determine a location of a potential failure event within the medical imaging system based on a) a signature in the signal received from the one or more sensors; and/or b) a difference between signals received from a plurality of the sensors.

## Description

### FIELD OF THE INVENTION

The present invention generally relates to medical imaging, and in particular relates to a defect localisation system and a computer-implemented method for determining a location of a potential failure event within a medical imaging system, and to a medical imaging arrangement.

### BACKGROUND OF THE INVENTION

Occasionally, a medical imaging system may fail to operate as expected. For example, arcing in a tube of an X-ray or CT imaging system may affect image quality, as well as workflow. Ultimately, arcing may result in a complete failure of the tube if it is not replaced in a timely manner. However, identifying a source of such defects can be time-consuming in view of the complexity of imaging systems. In the example of arcing, it is important to be able to ascertain whether an arc occurs in the tube, or in a power supply to the tube, in order that the correct replacement part can be ordered and a replacement carried-out efficiently. Additional knowledge of the nature of the arc may also be beneficial in determining a course of action. For example, a "hard" arc typically requires a scan to be aborted and that imaging is stopped at least for a period of time whilst the tube recovers. By contrast, a "soft" arc may permit scanning to continue.

### SUMMARY OF THE INVENTION

Thus, there may be a need to identify a location of a defect in a medical imaging system.

The object of the present invention is solved by the subject-matter of the independent claims, wherein further embodiments are incorporated in the dependent claims. It should be noted that the following described aspects of the invention apply also for the defect localisation system and the computer-implemented method for determining a location of a potential failure event within a medical imaging system, and the medical imaging arrangement.

According to a first aspect of the present invention, there is provided a defect localisation system for determining a location of a potential failure event within a medical imaging system. The defect localisation system comprises one or more sensors and at least one processing unit. The one or more sensors comprise at least one of an acoustic sensor, a vibration sensor, and a radio-frequency (RF) sensor. The at least one processing unit is configured to receive a signal generated by the one or more sensors. The at least one processing unit is further configured to determine a location of a potential failure event within the medical imaging system based on a) a signature in the signal received from the one or more sensors and/or b) a difference between signals received from a plurality of the sensors.

The defect localisation system as described herein performs defect localisation in a medical imaging system by measuring acoustic, vibrational, and/or RF signals that are characteristic of the defect using one or more sensors (e.g., acoustic, vibration, and/or RF sensors). Locating the source of the signal may be performed using an array of sensors based on e.g., relative amplitude and phase (e.g., phase-array or acoustic camera). An alternative or additional form of localisation is to classify key features in the waveform of the defect signal itself, which are characteristic of the signal source. The key features are also referred to as signatures of the signal, which may be detected using a single sensor (e.g., a single acoustic sensor, a single vibration sensor, or a single RF sensor). The signature of the signal may comprise one or more parameters (e.g., damping constant, natural resonance frequency, shape of the envelope, etc.) measured from the signal.

The defect localisation system as described herein may be suitable to remotely detect events and defects and to support root-cause identification. It may allow monitoring of systems and proactive action, even before the problem occurs to the users of the system. For example, in the case of soft-arcing it may be possible to prevent further deterioration or reoccurrence by so-called tube conditioning, or by changing settings in the system. In the case remote resolution of the problem is not possible anymore, the number of field service engineer's visits can be reduced and the amount of to-be-shipped spare-parts can be reduced. In addition, the medical imaging system experiences no (or less) unplanned downtime, in which patients cannot be scanned, thereby reducing planning (e.g., patient planning) problems in the hospital and improving overall efficiency.

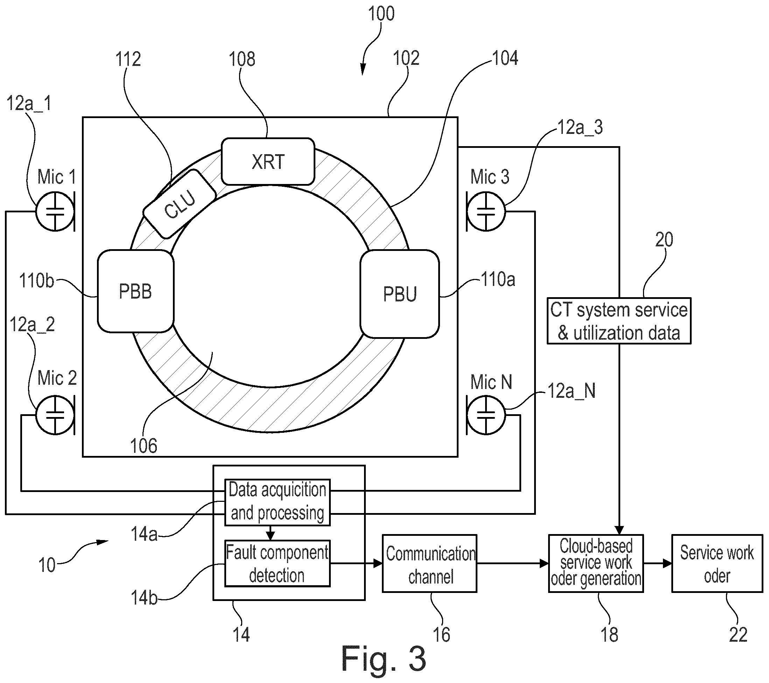

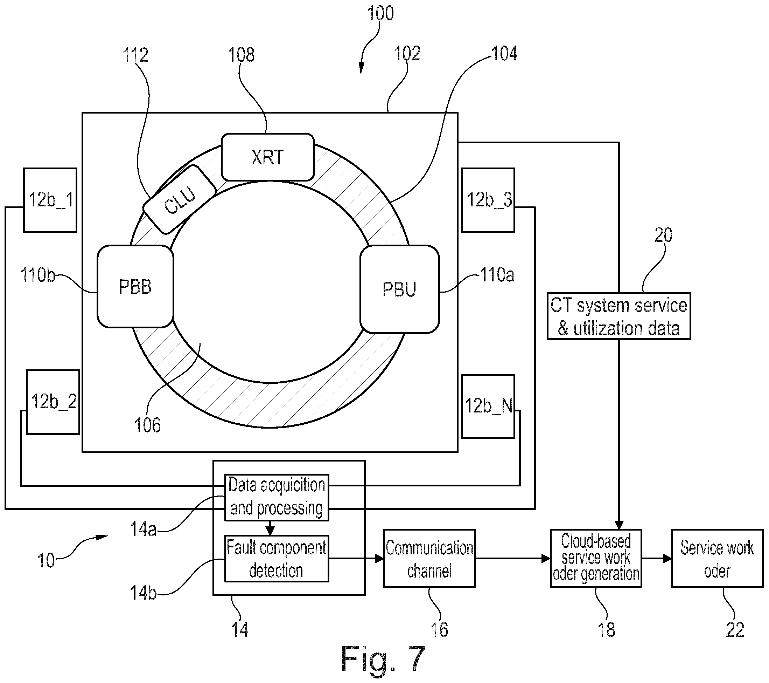

This will be described in detail hereinafter and in particular with respect to the example shown in Figs. 3 and 7.

According to an embodiment of the present invention, the one or more sensors are mountable at one or more locations on a stationary part of the medical imaging system and/or at one or more external locations in a room, in which the medical imaging system is disposed.

The one or more sensors are typically wired to more massive readout electronics. As the one or more sensors are attached to the fixed part instead of a moving part, such as rotating gantry, large centrifugal forces on this relatively heavy recorder part may be avoided. Thus, more complex components do not need to be protected against centrifugal forces.

This will be described in detail hereinafter and in particular with respect to the example shown in Fig. 3.

According to an embodiment of the present invention, the medical imaging system comprises a movable part. The potential failure event comprises a potential failure event on the movable part within the medical imaging system. The at least one processing unit is configured to receive a signal indicative of position information of the movable part within the medical imaging system and to determine a location of the potential failure event on the movable part within the medical imaging system based on the position information.

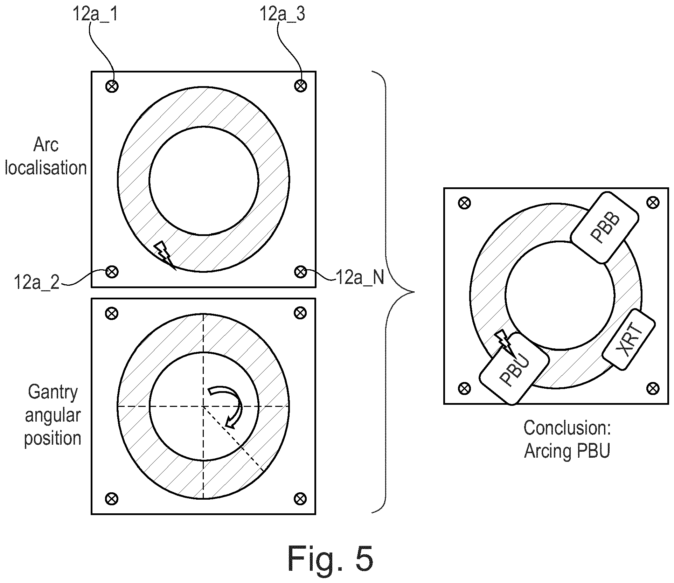

Some medical imaging systems may have failure events on the movable part. A complicating factor is that the movable part is performing linear and/or rotational motion during the failure event, e.g., gantry is rotating during the arcing event in a CT system. In such case, locating the source of the signal may be combined with position information of the (moving) object. The position of a moving object (e.g. a CT gantry) may be derived using the same sensor configuration either by making use of known sounds, vibrations, and/or RF signals coming from the moving object, and/or by adding sound generators "beacons" to this object. The combination of raw sensor signals and positioning information of the movable part may allow to uniquely identify the failure event on the movable part. This will be described in detail hereinafter and in particular with respect to the example shown in Fig. 5.

According to an embodiment of the present invention, the medical imaging system comprises a computed tomography, CT imaging system or an X-ray imaging system. The movable part within the medical imaging system comprises a rotatable gantry of the CT imaging system or a movable support of the X-ray imaging system.

According to an embodiment of the present invention, the signal indicative of position information of the movable part comprises a reading from a gantry or movable support position encoder.

For example, the at least one processing unit may perform simultaneous read-out of sensors and a gantry rotational encoder. By combining these two inputs, a unique failure event, e.g., arcing source, can be identified. This will be described in detail hereinafter and in particular with respect to the example shown in Fig. 3.

According to an embodiment of the present invention, the defect localisation system comprises at least one beacon that is attachable to the movable part, wherein the signal indicative of position information of the movable part comprises an audio, vibrational, and/or a radio-frequency signal generated by the at least one attached beacon.

In this embodiment, sound/vibration and/or RF generators may be provided to produce predetermined sound or RF patterns at specific positions on the gantry. These generators can act as 'beacons' for the sensor system that is located on the fixed world. The beacons can be used for calibration and they can be tracked by the sensor system to identify e.g., the rotational position of the gantry.

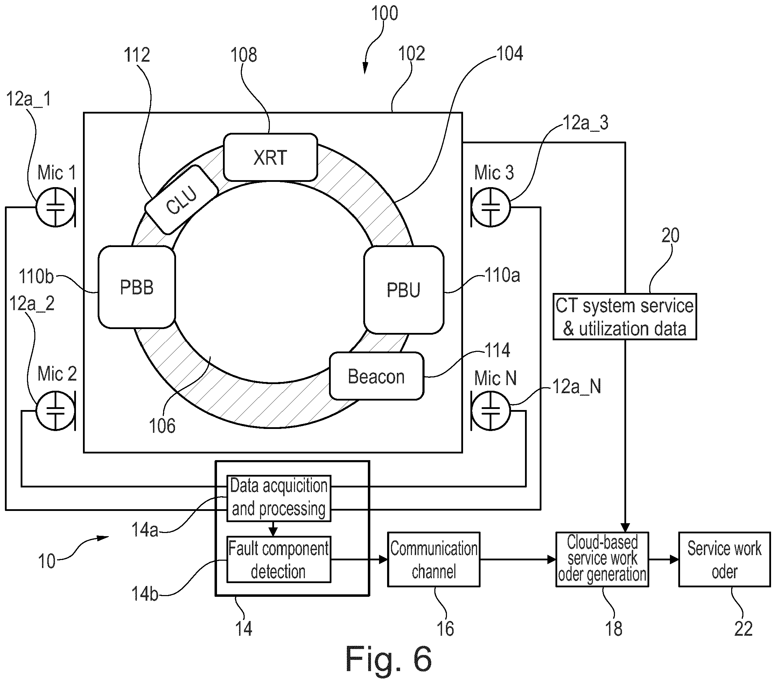

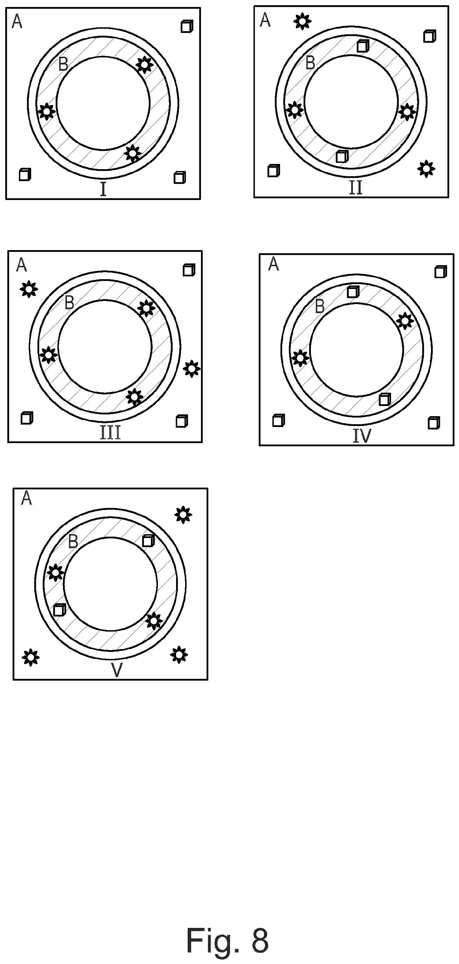

This will be described in detail hereinafter and in particular with respect to the example shown in Fig. 6. Further examples of the arrangement of the at least one beacon are illustrated in Fig. 8.

According to an embodiment of the present invention, the at least one beacon is provided with an acceleration sensor usable to provide sensor data indicative of a movable part spatial orientation. For example, the at least one beacon may be equipped with a three-dimensional (3D) acceleration sensor which allows to derive its position relative to the gravity of the earth irrespective of its own position and orientation on the gantry. Therefore, the time course of the 3D acceleration vector is recorded for several gantry rotations, resulting in a DC component due to the centrifugal force and a sinusoidal component due to gravity.

According to an embodiment of the present invention, the signal indicative of position information of the movable part comprises an audio, vibrational, and/or radio-frequency signal that is produced by a component of the movable part of the medical imaging system. The at least one processing unit is configured to identify the location of the potential failure event based on a known position of the component of the movable part within the medical imaging system with respect to the one or more sensors.

In this embodiment, it may denote a part already integrated in the moving/rotating part of the system which gets an additional function as beacon to generate a reference signal. In this way, no additional hardware integration and regulatory administration is needed for the beacon part. Examples of the component of the moveable part within the CT imaging system may comprise, but are not limited to, a rotating anode bearing, a fan, a pump, and a cooling system.

This will be described in detail hereinafter and in particular with respect to the example shown in Fig. 3.

According to an embodiment of the present invention, the potential failure event comprises one or more of: an arcing event, a worn component that causes noise and vibration, a mechanical component operating with changing noise signals or audio signals, a cooling fluid problem that generates characteristic noises upon gantry rotation, a contamination of a cooling fluid that causes a characteristic sound, and a thermal problem due to clogged filters in air circulation component.

According to an embodiment of the present invention, the difference between the signals received from the plurality of sensors comprises one or more of: a difference in amplitude between the signals received from the plurality of sensors, a difference in phase between the signals received from the plurality of sensors, and a difference in time of arrival between the signals received from the plurality of sensors.

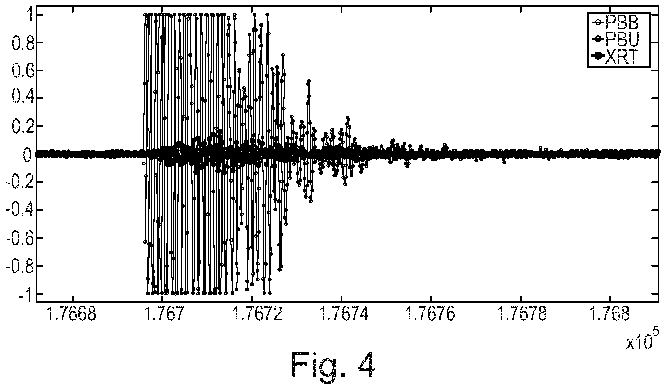

This will be described in detail hereinafter and in particular with respect to the example shown in Fig. 4.

According to an embodiment of the present invention, the defect localisation system further comprises a communication channel configured to connect the at least one processing unit to a remote service application. The at least one processing unit is configured to provide information about the detected potential failure event via the communication channel to the remote service application for generating a service work order and/or for training a predictive model for detecting a potential future system issue.

Exemplary information about the detected potential failure event may include, but is not limited to, the failure event identified, intermediate results of data processing, auxiliary and optionally raw sensor data (e.g., acoustic sensor data, vibration sensor data, RF sensor data, or any combination thereof).

For example, the information about the detected potential failure event may be used for remote diagnostics and first time right replacement of failed parts.

This will be described in detail hereinafter and in particular with respect to the example shown in Fig. 3.

According to an embodiment of the present invention, the service work order comprises one or more of automatic service scheduling, part ordering, and proposal of a change in a hospital's workflow.

According to an embodiment of the present invention, the at least one processing unit is configured to determine the location of the potential failure event within the medical imaging system based further on a model representing a relative location of the one or more sensors with respect to one or more components of the medical imaging system and/or one or more beacons.

According to a second aspect of the present invention, there is provided a medical imaging arrangement. The medical imaging arrangement comprises a medical imaging system; and a defect localisation system according to the first aspect and any associated example.

Examples of the medical imaging arrangement are shown in Figs. 3, 6 and 7.

According to a third aspect of the present invention, there is provided a computer-implemented method of determining a location of a potential failure event within a medical imaging system, the method comprising:
- receiving, by at least one processing unit, a signal generated by the one or more sensors, wherein the one or more sensors comprise at least one of an acoustic sensor, a vibration sensor, or a radio-frequency sensor; and
- determining, by the at least one processing unit, a location of a potential failure event within the medical imaging system based on:
   a) a signature in the signal received from the one or more sensors; and/or
   b) a difference between the signals received from a plurality of the sensors.

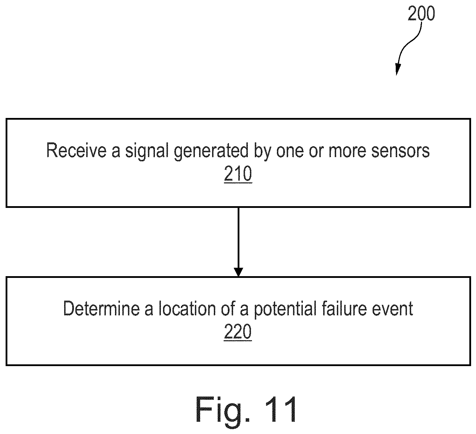

This will be described in detail hereinafter and in particular with respect to the example shown in Fig. 11.

According to a another aspect, there is provided a computer program product comprising instructions which, when the program is executed by a computer, cause the computer to carry out the steps of the method according to the third aspect and any associated example.

According to a further aspect, there is provided a computer-readable medium having stored thereon the computer program product.

As used herein, the terms "system", "unit", "module", etc., may include a hardware and/or software system that operates to perform one or more functions. For example, a module, unit, or system may include a computer processor, controller, and/or other logic-based device that performs operations based on instructions stored on a tangible and non-transitory computer readable storage medium, such as a computer memory. Alternatively, a module, unit, or system may include a hard-wired device that performs operations based on hard-wired logic of the device. Various modules, units, and/or systems shown in the attached figures may represent the hardware that operates based on software or hardwired instructions, the software that directs hardware to perform the operations, or a combination thereof.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig.. 1 schematically illustrates an exemplary medical imaging system.
Fig. 2 illustrates a transient plot of high voltage and related current in an X-ray system during arcing.
Fig. 3 illustrates an exemplary defect localisation system.
Fig. 4 illustrates sound sensor signals recorded at a position on the gantry.
Fig. 5 illustrates a concept of synchronization of the plurality of sensor-based measurements with the position information of the movable part within the medical imaging system.
Fig. 6 illustrates a further exemplary defect localisation system with at least one beacon.
Fig. 7 illustrates a further exemplary defect localisation system.
Fig. 8 illustrates seven examples I-VII of the sensor and beacon arrangement.
Fig. 9 illustrates an example of a transmitter circuit 24.
Fig. 10 illustrates an example of a receiver circuit 26.
Fig. 11 illustrates a flowchart describing an exemplary computer-implemented method.

### DETAILED DESCRIPTION OF EMBODIMENTS

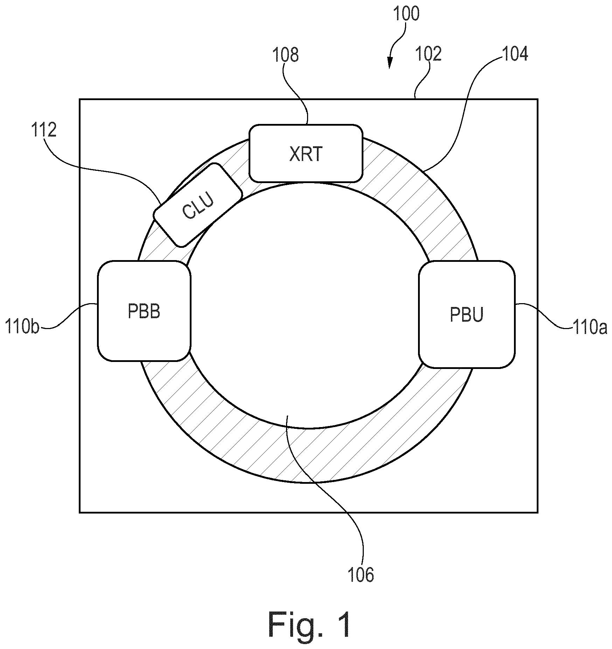

Fig. 1 schematically illustrates an exemplary medical imaging system 100 that may be suitable for implementation of the present approach. In the illustrated example, the exemplary medical imaging system 100 is a CT imaging system. The CT imaging system 100 may be utilized to perform diagnostic scans of a region of interest of a human subject (not shown).

In the illustrated example, the CT imaging system 100 includes a non-rotating gantry 102, a rotating gantry 104, and a bore 106 that defines an examination region of the CT imaging system. An X-ray system is used on the rotating gantry 104. The X-ray system includes an X-ray tube (XRT) 108 as a source, an X-ray detector (not shown) mounted opposite on the rotating gantry 104 to receive radiation from the X-ray tube 108 after the X-rays transverse the examination region, and a high-voltage generation system 110. The latter may be potentially decomposed in multiple building blocks, such as power block unit (PBU) 110a and power block booster (PBB) 110b. The different sub-elements are connected via high-voltage cables (not shown). A cooling unit (CLU) 112 may be provided to remove the heat from the critical parts of the system.

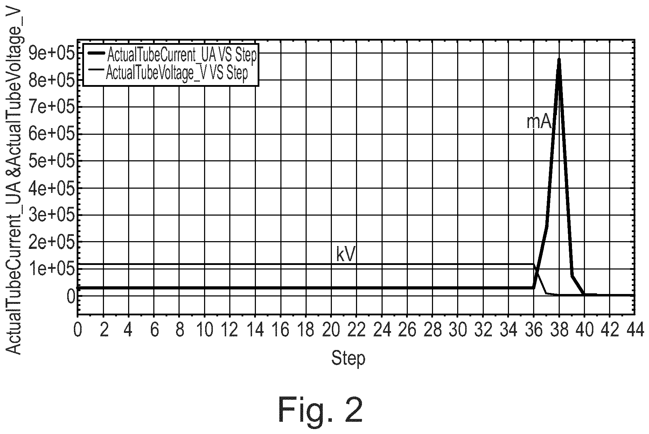

With the use of high voltages in the system a risk of arcing is introduced. This arcing may take place at different locations in the system, like in the XRT, in the PBU, in the PBB, or at the different cables and connectors. During arcing the isolation (e.g., air, partial vacuum, or insulating material) may break down and an avalanche of ionisation may cause large currents to flow and the high voltage to collapse. For example, Fig. 2 illustrates a transient plot of high voltage and related current in an X-ray system during arcing. When arcing occurs, the current suddenly increases and the high-voltage collapses as it cannot accommodate the increased load. In such case, the system may get out of control and proper X-ray generation and imaging is not possible. This means that at least part of the data that is needed for three-dimensional image reconstruction is missing. Moreover, the arcing may cause damage to the different components in the system (e.g., connector insulators showing conducting carbon tracks), which will then need replacement. For this reason, the arcing is highly unwanted and may cause the overall system to go down with large impact on hospital efficiency and planning, (e.g., patient planning).

Common causes of arcing may include outgassing of the X-ray vacuum tube, leakage in that same tube, high field strengths in the power block components, or contamination at the cable connector positions. Generally, a field service engineer (FSE) has to go onsite to resolve the problem by replacing the arcing component. Finding the component that causes the arcing is not straightforward. Generally, it is done by replacing parts (e.g., field replaceable units) one-by-one in a trial-and-error procedure. This means that an arcing problem has severe financial and non-financial consequences. For example, the CT system experiences unplanned downtime, in which patients cannot be scanned, leading to planning (e.g., patient planning) problems in the hospital and lower overall efficiency. Costly and lengthy visits of FSE's are required to resolve the issue, in particular to identify the arcing component. Unnecessary replacement of components leads to significant part restocking, which includes costly waste of parts. As a result of the above customer satisfaction may drop causing customer retention and service contract renewal rate to be at risk.

Towards this end, a defect localisation system is provided for determining a location of a potential failure event within a medical imaging system, such as arcing localisation on a rotating gantry of a CT imaging system. The defect localisation system comprises one or more sensors and at least one processing unit. The at least one processing unit is configured to receive a signal generated by the one or more sensors, and to determine a location of a potential failure event within the medical imaging system based on a) a signature in the signal received from the one or more sensors and/or b) a difference between signals received from a plurality of the sensors.

In some examples, it is possible that the formation of some potential failure events, such as an electric arc, will occur simultaneously with the emission of the recognizable acoustic waves. In these examples, failure event detection may be made possible via the detection of acoustic waves using one or more acoustic sensors.

In some examples, the emergence of the potential failure event, such as a worn component, may cause vibration in the system. In these cases, the measurement of vibration using one or more vibration sensors, such as an accelerometer, encoder, etc., may be used to identify these failure events.

In some examples, some potential failure events, such as cooling fluid problem, may generate characteristic RF noises upon gantry rotation in the system. In these cases, the measurement of RF noise using one or more RF sensors may be used to identify these failure events.

In order to detect one or more of these potential failure events, the one or more sensors may comprise one or more acoustic sensors, one or more vibration sensors, one or more RF sensors, or any combination thereof.

Fig. 3 illustrates an exemplary defect localisation system 10 for determining a location of a potential failure event within a medical imaging system, e.g., the CT imaging system 100 shown in Fig. 1. The defect localisation system 10 may be implemented in several ways. In some examples, the defect localization system may be an integral (e.g., designed-in) part of the system. Alternatively, the defect localization system 10 may only be a separate part of the system. The defect localization system may even be installed at a later moment when it is designed as a completely independent and self-supporting sensor system that has its own power supply and own cloud connection.

In the following, arcing localisation on a rotating gantry of a CT imaging system is described for the implementation of the present approach by way of example. However, it will be appreciated that the defect localisation system as described herein is also applicable for other types of failure event detection. For example, the defect localisation system as described herein may be applicable for detecting and localising a worn component that causes noise and vibration. For examples, the defect localisation system as described herein may be applicable for detecting and localising a mechanical component operating with changing noise signals or audio signals due to one or more of changing environmental conditions, changing temperature condition, changing subcomponent aging condition, changing location, and/or grid voltage variation. For examples, the defect localisation system as described herein may be applicable for detecting and localising a cooling fluid problem that generates characteristic noises upon gantry rotation. For example, the defect localisation system as described herein may be applicable for detecting and localising a contamination of a cooling fluid that causes a characteristic sound. For example, the defect localisation system as described herein may be applicable for detecting and localising a thermal problem due to clogged filters in air circulation component. In other words, the defect localisation system as described herein is not limited to arcing localisation only, but may also be used to detect and monitor other problems of an imaging or other kind of system. This can include mechanical problems (e.g., worn components that causes noise and vibration), cooling oil problems (e.g., air bubbles that generate characteristic noises upon gantry rotation), thermal problems due to clogged filters in air circulation components, etc.

The exemplary defect localisation system 10 comprises one or more sensors 12 and at least one processing unit 14.

The one or more sensors 12 comprise one or more acoustic sensors 12a, e.g., acoustic sensors 12a_1, 12a_2, 12a_3, ..., 12a_N shown in Fig. 3, to detect acoustic waves in the system. Although four acoustic sensors are illustrated in Fig. 3 by way of example, in alternative embodiments, a different number of acoustic sensors, such as one, two, three, five, six, or more acoustic sensors, may be provided to detect acoustic waves in the system.

The one or more acoustic sensors 12a may have an operating frequency range covering the frequency of the acoustic waves caused by the potential failure events, e.g., typical emission frequencies of the electric arcs. In some examples, the one or more acoustic sensors 12a may have an operating frequency range of tens of millihertz (mHz) for some applications. In some examples, the one or more acoustic sensors 12a may have an operating frequency range of several megahertz (MHz) for the ultrasonic applications.

The one or more acoustic sensors 12a may be arranged on a fixed part. In some examples, the one or more acoustic sensors 12a are mountable, such as removably mounted (e.g., using a mechanical holder) or integrated, at one or more locations on a stationary part of the medical imaging system 100. For example, the acoustic sensors 12a_1, 12a_2, 12a_3, ..., 12a_N shown in Fig. 3 may be integrated in the non-rotating gantry 102 of the medical imaging system 100. In some examples, the one or more acoustic sensors 12a may be mountable, such as removably mounted or integrated, at one or more external locations in a room (e.g., examination room), in which the medical imaging system 100 is disposed.

The one or more acoustic sensors 12a detect acoustic waves within the medical imaging system 100 and provide output signals to the at least one processing unit 14. The output signals of the one or more acoustic sensors 12a may be analogue signals and/or digital signals.

The at least one processing unit 14 may comprise various physical and/or logical components for communicating and manipulating information, which may be implemented as hardware components (e.g. computing devices, processors, logic devices), executable computer program instructions (e.g. firmware, software) to be executed by various hardware components, or any combination thereof, as desired for a given set of design parameters or performance constraints. In some implementations, the at least one processing unit 14 may comprise one or more microprocessors or computer processors, which execute appropriate software. The software may have been downloaded and/or stored in a corresponding memory, e.g. a volatile memory such as RAM or a non-volatile memory such as flash. The software may comprise instructions configuring the one or more processors to perform the functions described herein. It is noted that the at least one processing unit may be implemented with or without employing a processor, and also may be implemented as a combination of dedicated hardware to perform some functions and a processor (e.g. one or more programmed microprocessors and associated circuitry) to perform other functions. For example, the at least one processing unit may be implemented in the device or apparatus in the form of programmable logic, e.g. as a Field-Programmable Gate Array (FPGA). In some examples, the at least one processing unit 14 may be embodied in a system controller of the medical imaging system 100. In some other examples, the at least one processing unit may be embodied as, or in, other devices, e.g., a workstation.

In some examples, as shown in Fig. 3, the at least one processing unit 14 may comprise a data acquisition and processing unit 14a and a fault component detection unit 14b. The data acquisition and processing unit 14a may allow on-site sensor signal pre-processing, e.g., signal filtering, signal transformation, feature extraction, and data reduction. As an example, the data acquisition and processing unit 14a may perform a band-pass filtering of the output signals of the acoustic sensors 12a_1, 12a_2, 12a_3, ..., 12a_N based on the typical emission frequencies of the electric arcs.

Following the initial processing, the fault component detection unit 14b may perform any suitable algorithm to perform fault detection. In some examples, the fault component detection unit 14b may determine a location of a potential failure event within the medical imaging system based on a signature in the signal received from the one or more acoustic sensors 12a. The algorithm may be an algorithm for "signature" or "fingerprint" detection, such as correlation-based signal "fingerprint" detection. The so-called signal "signature" or "fingerprint" detection is a method to identify the origin of the arcing. Fig. 4 illustrates sound sensor signals recorded at a position on the gantry. In Fig. 4 it is clearly observable that the sound or vibration signal behaves as a damped oscillation. Exemplary key signal features, such as damping constant, natural resonance frequency, and/or shape of the envelope (e.g., ring down behaviour), may depend on the component causing the arcing and the components near to it. Therefore, a signal processing algorithm (e.g., classifier) may be implemented to enable arcing localisation. In some examples, the "signature" detection, e.g., the detection of damping constant, natural resonance frequency, and/or shape of the envelope, may be performed based on conventional signal processing techniques using filtering and signal transformations (e.g., Fourier transforms). In some other examples, the "signature" detection may be done by intelligent algorithms using artificial intelligent networks and machine learning techniques.

In some examples, the fault component detection unit 14b may determine a location of a potential failure event within the medical imaging system based on a difference between signals received from a plurality of the acoustic sensors 12a, e.g., acoustic sensors 12a_1, 12a_2, 12a_3, ..., 12a_N shown in Fig. 3. Examples of the difference between signals received from the plurality of the acoustic sensors 12A, 12B, 12C, and 12D will be described in detail below.

In a first example, the difference between the signals received from the plurality of acoustic sensors 12a may comprise a difference in amplitude between the signals received from the plurality of sensors. In other words, relative amplitude may be an indication of the arcing position. As can be seen in Fig. 4, the PBB signal is significantly larger compared to the other two signals. Therefore, it is highly likely that the arcing occurred in or close to the PBB.

In a second example, the difference between the signals received from the plurality of acoustic sensors 12a may comprise a difference in phase between the signals received from the plurality of sensors. In other words, the relative phase may be used to perform arcing localisation. The sound/vibrations travel through air and/or the system components with a speed of few hundred to a few thousand meters per second, which may depend on the mechanical compliance properties of the sound-conducting material. With multiple acoustic sensors 12a, the algorithm may perform triangulation or even more sophisticated phased-array or "acoustic camera" methods. Focused `listening' is possible using beamforming techniques where different signals are added with suitable delays. This method may enable the system to be robust against background noises and sounds. When normalizing the signals in Fig. 4, one can clearly observe that the signal waveform originating from the PBB starts earlier compared to the other two signal waveforms. This is another indication that arcing occurred in or close to the PBB.

In a third example, the difference between the signals received from the plurality of acoustic sensors 12a may comprise a difference in time of arrival between the signals received from the plurality of sensors. For example, the acoustic sensors 12a may receive the acoustic signal caused by the potential failure events, e.g., arcing event, and the Time Difference on Arrival (TDOA) approach may be utilized to look at the difference in arrival time of the acoustic signal between each acoustic sensor 12a to localize the component causing the arcing.

A complicating factor may be that some medical imaging systems may comprise a movable part with e.g., a linear and/or rotational motion, and the potential failure event may comprise a potential failure event on the movable part within the medical imaging system. For example, in the illustrated medical imaging system 100 shown in Fig. 3, the movable part within the medical imaging system comprises the rotatable gantry 104. In other X-ray imaging systems (not shown), the movable part within the medical imaging system may comprise a movable support of the X-ray imaging system. This means that even if the source of the sound is located, the arcing generating component on the movable part, e.g., rotating gantry 104 of the illustrated example, cannot be uniquely identified as the positioning information of the movable part at the moment of failure event, e.g., rotational position of the gantry at the moment of arcing, is not known.

Towards this end, the at least one processing unit 14 may be configured to provide synchronization of the plurality of sensor-based measurements with the position information of the movable part, thereby allowing to uniquely identify the fault component on the movable part. The concept is shown in Fig. 5. In the illustrated example, the at least one processing unit may determine the arc localisation based on the sensor signals received from the acoustic sensors 12a, e.g., acoustic sensors 12a_1, 12a_2, 12a_3, ..., 12a_N shown in Fig. 5. The at least one processing unit 14 may also receive a signal indicative of position information of the movable part within the medical imaging system, e.g., the gantry angular position *θ* shown in Fig. 5. The at least one processing unit 14 may be configured to provide synchronization of the plurality of sensor-based measurements with CT gantry angular position, thereby allowing to uniquely identify the arcing generating component (e.g., the PBU shown in Fig. 5) on the rotating gantry.

The at least one processing unit 14 may receive one or more of the following exemplary signals to determine the position information of the movable part.

In a first example, the signal indicative of position information of the movable part may comprise a reading from a gantry or movable support position encoder. For example, the at least one processing unit 14 shown in Fig. 3 may perform simultaneous read-out of acoustic sensors and a gantry rotational encoder. By combining these two inputs, the at least one processing unit 14 may identify a unique arcing source. In this example, no additional hardware integration and regulatory administration is needed for the beacon part. However, the at least one processing unit 14 needs access to the rotational encoder. For third-party systems the rotational encoder information may not even be accessible. This may complicate the design or integration of this solution, and make this solution not vendor agnostic.

In a second example, the signal indicative of position information of the movable part may comprise an audio, vibrational, and/or RF signal that is produced by a component of the movable part of the medical imaging system. The at least one processing unit 14 may be configured to identify the location of the potential failure event based on a known position of the component of the movable part within the medical imaging system with respect to the one or more sensors. As an example, the at least one processing unit 14 may derive the gantry rotation from the acoustic signals measured by the acoustic sensors 12a, e.g., acoustic sensors 12a_1, 12a_2, 12a_3, ..., 12a_N shown in Figs. 3 and 5. These acoustic signals may be generated by one or more equipment installed on known locations on the gantry. For example, in a CT system, some equipment, e.g., the rotating anode bearing, fans, pump, cooling system, etc., may produce characteristic sound, vibrational, and/or RF signals. Using the same signal processing techniques e.g., based on relative amplitude, phase, etc., the at least one processing unit 14 may determine the position of these equipment at any moment in time and in this way derive information on the rotational position of the complete gantry. In this example, a part already integrated in the moving/rotating part of the system may get an additional function as beacon. In this way, no additional hardware integration and regulatory administration is needed for the beacon part. Additionally, the arcing localisation system knows the absolute gantry position without requiring access to the gantry rotational encoder.

In a third example, as shown in Fig. 6, at least one beacon 114 is provided that is attachable to the movable part, e.g., the rotatable gantry 104 of the medical imaging system 100 shown in Fig. 6. The signal indicative of position information of the movable part comprises an audio signal, a vibrational signal, and/or an RF signal generated by the at least one attached beacon 114. In other words, the beacon may denote an extra component added to generate a reference signal. Although one beacon is illustrated in Fig. 6 by way of example, in alternative embodiments, a plurality of beacons may be provided. In other words, one or more acoustic generators, vibration generators, and/or RF generators may be provided that produce predetermined acoustic, vibrational, and/or RF patterns at specific positions on the gantry. The one or more generators may act as 'beacons' for the sensor system that is located on the fixed world. In the example of Fig. 6, the at least one beacon may be an acoustic generator for the acoustic sensor system, i.e., the acoustic sensors 12a_1, 12a_2, 12a_3, ..., 12a_N shown in Fig. 6. The at least one beacon may be used for regular or irregular calibration, and can be tracked by the sensor system e.g., the acoustic sensors 12a, again using signal processing techniques as described above to identify the rotational position of the gantry. In some examples, if one or more acoustic beacons are used, the acoustic beacon(s) may have frequency spectra outside the hearing range or are sufficiently low in intensity to avoid any annoyance to the user. In some examples, the at least one beacon 114 may be equipped with a 3D acceleration sensor which allows to derive its position relative to the gravity of the earth irrespective of its own position and orientation on the gantry. Therefore, the time course of the 3D acceleration vector may be recorded for several gantry rotations, resulting in a DC component due to the centrifugal force and a sinusoidal component due to gravity. Fitting of a respective model may allow to derive the exact angular position of the at least one beacon relative to gravity/the vertical direction at any time and also the angular rotation speed of the gantry. The at least one beacon 114 may encode this data in real time into the signal that is emitted, e.g., by frequency modulation of the emitted signal. In some examples, the angular position may be directly encoded in the frequency using a sawtooth modulation. More sophisticated and also digital encodings may be used. This acceleration concept may have the advantage that the arcing localisation system knows the absolute gantry position without requiring access to the gantry rotational encoder. The above-described exemplary set-up with acoustic sensors on the fixed part (e.g., attached to the medical imaging system and/or next to the medical imaging system in the examination room) with at least one beacon on the rotating gantry may also have the advantage that only the more compact and wireless beacons have to be attached to the gantry, while the sensors typically wired to more massive readout electronics can be much more easily fixed on the stator. So, these more complex components do not need to be protected against centrifugal forces of up to 6g.

Turning back to Fig. 3, a communication channel 16 may be provided to connect the at least one processing unit 14 to a remote service application different from the communication channel system under monitoring utilized for reporting its service data to its remote service application. As shown in Fig. 3, the remote service application may be executed in the cloud for cloud-based service work order generation. In some other examples (not shown), the remote service application may be executed on hospital premises.

In some examples, the at least one processing unit 14 may be configured to provide information about the detected potential failure event via the communication channel 16 to the remote service application 18 for generating a service work order 22. Exemplary information about the detected potential failure event may include, but is not limited to, the failure identified, intermediate results of data processing, auxiliary and optionally raw sensor data (e.g., acoustic sensor data, vibration sensor data, RF sensor data, or any combination thereof). In some examples, the cloud-based service work order generation may combine the sensor-based fault information generated by the at least one processing unit 14 with other CT system service and utilization data obtained from the medical imaging system 100 to generate a proper service work order based on the data received from the local processing unit, e.g., the at least one processing unit 14. For example, the service work order may comprise automatic service scheduling for a field service engineer, service team, etc. in case of failure occurrence or need of predictive maintenance. For example, the service work order may comprise part ordering for an about-to-fail part replacement. In some examples, the service work order may comprise a proposal of a change in a hospital's workflow, and a preventive maintenance schedule can be set with the customer to reduce the down time. This may help to reduce the downtime of the medical device and upholds the customer satisfaction.

In some examples, the at least one processing unit 14 may be configured to provide information about the detected potential failure event via the communication channel 16 to the remote service application 18 for training a predictive model for detecting a potential future system issue. For example, the communications channel 16 to the cloud may allow to offload and store massive amounts of additional sensor signal data, e.g., raw sensor signal data, from a large installed base of the medical imaging system in many different environments. The data can be used to further improve fault-detection algorithms in the future, which means to enable predictive model training based on the data received from the local processing unit (e.g., the at least one processing unit 14) and the system service and utilization data 20 from the medical imaging system under monitoring received via a system service communication channel.

Fig. 7 illustrates a further exemplary defect localisation system 10 for determining a location of a potential failure event within a medical imaging system. The illustrated exemplary defect localisation system 10 is similar to the exemplary defect localisation system 10 shown in Fig. 3 with the exception of the sensors 12 comprising one or more vibration sensors 12b, e.g., vibration sensors 12b_1, 12b_2, 12b_3,... 12b_N shown in Fig. 7. Although four vibration sensors are illustrated in Fig. 7 by way of example, in alternative embodiments, a different number of acoustic sensors, such as one, two, three, five, six, or more acoustic sensors, may be provided to identify failure events. Examples of the vibration sensors 12b may include, but are not limited to, accelerometer, potentiometer, and encoder. The vibration sensors 12b may be utilized for detecting and localising a fault component, e.g., a worn component, which causes noise and vibration. The one or more vibration sensors 12b may be mountable, such as removably mounted or integrated, at one or more locations on a stationary part (e.g., non-rotating gantry 102) of the medical imaging system 100 for vibration detection. The vibration sensors 12b provide output signals to the at least one processing unit 14.

The at least one processing unit 14 may perform a similar function as the at least one processing unit described with respect to the example shown in Fig. 3. For example, the at least one processing unit 14 may perform simultaneous read-out of the vibration sensors and the gantry rotational encoder to allow to uniquely identify the failure event. For example, the at least one processing unit 14 may derive the gantry rotation from the vibration signal produced by a component of the movable part of the medical imaging system. Although not shown in Fig. 7, in alternative embodiments, one or more beacons may be provided that are attachable to the movable part, e.g., the rotatable gantry 104 of the medical imaging system 100 shown in Fig. 7. In other words, the one or more beacons may denote an extra component added to generate a reference signal. The one or more beacons may generate a vibration signal to be tracked by the one or more vibration sensors 12b. The exemplary signal processing techniques as described above may be used to identify the rotational position of the gantry.

In some other examples (not shown), the one or more sensors may comprise one or more RF sensors for detecting and localising a potential failure event that causes an RF noise. The one or more RF sensors may have an operating frequency range that is dependent on the RF noise caused by the potential failure event. The RF sensors may be arranged on a fixed part. In some examples, the RF sensors are mountable, such as removably mounted or integrated, at one or more locations on a stationary part (e.g., non-rotating gantry 102) of the medical imaging system 100. In some examples, the RF sensors may be mountable, such as removably mounted or integrated, at one or more external locations in a room (e.g., examination room), in which the medical imaging system 100 is disposed. The RF sensors provide output signals to the at least one processing unit. The at least one processing unit may performs a similar function as the at least one processing unit 14 described with respect to the example shown in Fig. 3. For example, the at least one processing unit may perform simultaneous read-out of the RF sensors and the gantry rotational encoder to allows to uniquely identify the failure event. For example, the at least one processing unit may derive the gantry rotation from the RF signal produced by a component of the movable part of the medical imaging system. In some examples, one or more beacons may be provided that are attachable to the movable part, e.g., the rotatable gantry 104 of the medical imaging system 100 to denote an extra component added to generate a reference signal. The one or more beacons may generate an RF signal to be tracked by the RF sensors. The exemplary signal processing techniques as described above may be used to identify the rotational position of the gantry.

In some further examples, the one or more sensors may comprise any combination of the above-described sensors, such as acoustic sensors, vibration sensors, and RF sensors, for detecting and localising different types of failure events.

In Fig. 6 the at least one beacon is provided on the rotating gantry and the sensors are provided on a fixed part (e.g., on the non-rotating gantry). In some other examples, the sensors may be provided on the rotating gantry and beacons may be provided on the non-rotating gantry, and also any mixed set-up. This may have the advantage that sensor(s) may be directly attached to parts known or prone to arcing for improved signal-to-noise ratio (SNR). Fig. 8 illustrates seven examples I-VII of the sensor and beacon arrangement, where A denotes the stationary part and its vicinity, and B the moving (e.g., rotating) part. The black sun symbol denotes a beacon and the black box symbol denotes a sensor.

As shown in example I, the beacon(s) may be arranged on the moving part (e.g., rotating gantry), and the sensor(s) may be arranged on the stationary part (e.g., non-rotating gantry).

As shown in example II, a plurality of beacons may be provided and arranged on the moving part (e.g., rotating gantry) and on the stationary part (e.g., non-rotating gantry). In addition, a plurality of sensors may be provided and arranged on the moving part (e.g., rotating gantry) on the stationary part (e.g., non-rotating gantry).

As shown in example III, a plurality of beacons may be provided and arranged on the moving part (e.g., rotating gantry) and on the stationary part (e.g., non-rotating gantry). In addition, a plurality of sensors may be provided and arranged on the stationary part (e.g., non-rotating gantry).

As shown in example IV, a plurality of beacons may be provided and arranged on the moving part (e.g., rotating gantry). In addition, a plurality of sensors may be provided and arranged on the moving part (e.g., rotating gantry) and on the stationary part (e.g., non-rotating gantry).

As shown in example V, a plurality of beacons may be provided and arranged on the moving part (e.g., rotating gantry) and on the stationary part (e.g., non-rotating gantry). In addition, a plurality of sensors may be provided and arranged on the moving part (e.g., rotating gantry).

Although this application describes a CT system for the implementation of the present approach by way of example, it will be appreciated that the described fault detection is not limited to a rotating gantry but it can be used for any moving object where it is desirable to locate an adverse event. Thus, the present approach is applicable to many system with linear and/or rotational motion. In addition, the defect localisation system is not limited to arcing localisation only, but may also be used to detect and monitor other problems of an imaging or other kind of system. This can vary from mechanical problems (e.g., worn components that causes noise and vibration), cooling oil problems (e.g., air bubbles that generate characteristic noises upon gantry rotation), thermal problems due to clogged filters in air circulation components. Furthermore, it can also be used to monitor system usage. The defect localisation system as described herein may also be used for e.g. image-guided therapy (IGT) systems and other X-ray systems with moving or distributed high voltage components. Therefore, the defect localisation system as described herein is applicable for multi-purpose failure or status detection, e.g., for modalities other than CT, such as C-arm, single-photon emission computed tomography (SPECT) systems, and combinations thereof with mechanically moving parts.

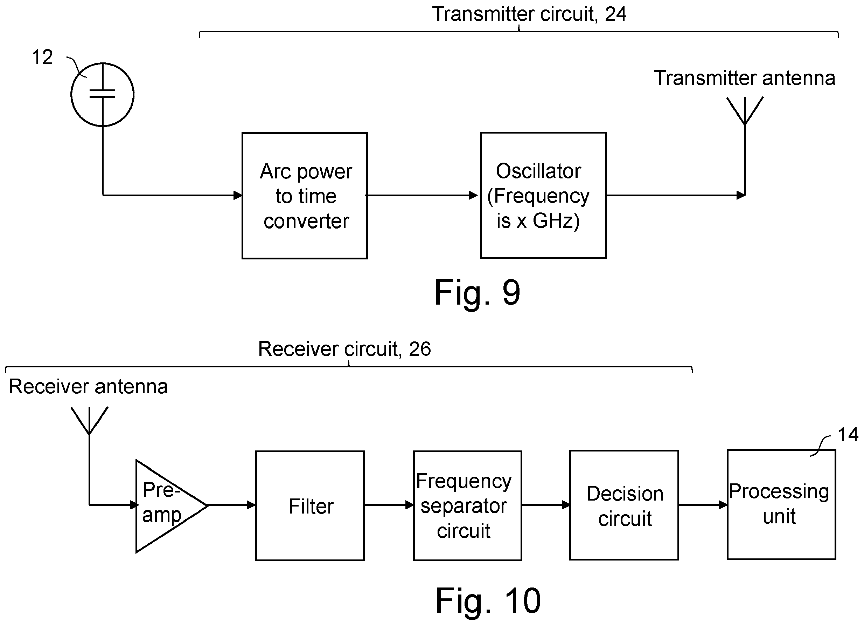

In one example, the defect localisation system is configured to determine a location of a potential failure event within a medical imaging system, and the potential failure event is an arcing event. In this example, each sensor is provided with a transmitter circuit, and the transmitter circuit transmits encoded signals for the corresponding sensor. This example is described with reference to Fig. 9 and Fig. 10.

In this example, the defect localisation system comprises a plurality of the sensors 12, and each sensor further comprises a transmitter circuit 24, the transmitter circuit being configured to transmit encoded signals for the sensor 12, the encoded signals representing i) the signal generated by the sensor 12 in response to a detection of the arcing event by the sensor, and ii) an identity of the sensor 12. The defect localisation system also includes a receiver circuit 24, the receiver circuit being configured to: receive the encoded signals transmitted by the transmitter circuits;
extract from the encoded signals: i) the signal generated by the sensor in response to the detection of the arcing event by the sensor, and ii) the identity of the sensor; and
communicate to the at least one processing unit (14): i) the extracted signal generated by the sensor in response to the detection of the arcing event by the sensor, and ii) the extracted identity of the sensor.

In this example, the at least one processing unit (14) is further configured to receive the identity of the sensor for the signals generated by the sensors, and to determine the location of the arcing event within the medical imaging system based further on c) the identities of the sensors from which the signals are received.

An example of a transmitter circuit 24 in accordance with this example is illustrated in Fig. 9. An example of the receiver circuit 26 in accordance with this example is illustrated in Fig. 10. With reference to the example transmitter circuit 24 illustrated in Fig. 9, in this example the sensor 12 is provided by an acoustic sensor. However, more generally, the sensor 12 may be provided by any of the aforementioned types of sensors, i.e. an acoustic sensor, a vibration sensor, and a radio-frequency (RF) sensor. In this example, the sensors and their corresponding receiver circuits are distributed in various positions around the medical imaging system. A sensors and its corresponding receiver circuit may be arranged on a moving part of the medical imaging system (e.g., on a rotating gantry). Alternatively, a sensors and its corresponding receiver circuit may be arranged on a stationary part of the medical imaging system. Alternatively, a sensor and its corresponding receiver circuit may be arranged at one or more external locations in a room, in which the medical imaging system is disposed.

When an arc occurs, a signal is generated by the sensor 12, and the transmitter circuit 24 transmits encoded signals to the receiver 26. The encoded signals represent: i) the signal generated by the sensor in response to the detection of the arcing event by the sensor, and ii) the identity of the sensor. Various elements of the signal generated by the sensor in response to the detection of the arcing event by the sensor, may be represented in the encoded signals that are transmitted to the receiver 26. For instance, the generated signal itself may be encoded and transmitted. Alternatively or additionally, the time of generation of the signal by the sensor in response to the detection of the arcing event by the sensor, may be encoded and transmitted. Alternatively or additionally, the energy, or the amplitude, of the signal generated by the sensor in response to the detection of the arcing event by the sensor, may be encoded and transmitted. The energy of the signal generated by the sensor corresponds to the detected energy of the arc. The energy of the signal generated by the sensor may be determined by integrating the signal generated by the sensor in response to the detection of the arcing event by the sensor. Alternatively or additionally, other elements of the signal such as the rate of change of the signal may be encoded, and transmitted in a similar manner. The identity of the sensor is also encoded and transmitted to the receiver 26. The identity of the sensor may be any identifier that uniquely identifies the corresponding sensor. For instance, the identity may be a number, or a code.

The advantage of determining the location of the arcing event based on the encoded signals in this manner is to remove interference and also interruptions to the signals in the vicinity of the medical imaging system. The encoding that is performed by the transmitter circuit 24 may be performed using various techniques, such as by modulating a carrier frequency of a signal that is emitted by the transmitter circuit, or by embedding a digital code into a signal that is emitted by the transmitter circuit. By way of an example, in the example illustrated in Fig. 9, the encoded signals represent i) the energy of the signal generated by the sensor in response to the detection of the arcing event by the sensor, and ii) the identity of the sensor. In this example, the energy of the signal generated by the sensor is determined by converting the detected power of the arc to a period of time in the unit "Arc to time converter", and transmitting a carrier frequency (in the example, at x GHx) for the corresponding period of time. A different carrier frequency, i.e. x, is used for each transmitter circuit, and this consequently represents the identity of the sensor. The advantage of performing localisation using this period of time instead of e.g. the time difference in detecting the arc between different sensors is two-fold. Firstly, the signal is more distinct and therefore less affected by surroundings. Secondly, there is no need to synchronise the different receiver circuits with a common clock.

Instead of transmitting the energy of the signal generated by the sensor, the signal itself may be transmitted by e.g. using the signal to perform a frequency modulation of the carrier frequency. Instead of transmitting the energy of the signal generated by the sensor, the amplitude of the signal generated by the sensor may be transmitted, in this case by again converting the amplitude to time, and transmitting the carrier frequency for the corresponding period of time.

In some examples, the transmitter circuit 24 is a wireless, (i.e. RF) transmitter circuit. For instance, the transmitter circuit 24 illustrated in Fig. 9 uses an antenna to wirelessly transmit the encoded signals to the receiver circuit 26. The energy consumed by the wireless transmitter circuit 24 may be provided by a battery. Alternatively, the energy may be harvested from detected RF signals. The transmitter circuit 24 may alternatively use other another form of communication to communicate the encoded signals to the at least one processing unit 14. For instance, an optical fiber, or electrical wires may be used. The transmitter circuit 24 may include a filter and/or an amplifier in order to respectively filter and/or amplify the signal generated by the sensor, and to thereby remove excess frequencies/ improve signal to noise ratio of the encoded signals.

The receiver circuit 26 illustrated in Fig. 10 receives the encoded signals that are transmitted by the transmitter circuits. The receiver circuit 26 also extracts from the encoded signals: i) the signal generated by the sensor in response to the detection of the arcing event by the sensor, and ii) the identity of the sensor. The receiver circuit 26 also communicates to the at least one processing unit (14): i) the extracted signal generated by the sensor in response to the detection of the arcing event by the sensor, and ii) the extracted identity of the sensor. In the illustrated example, the receiver circuit includes a receiver antenna that detects wireless (i.e. RF) encoded signals. In the illustrated example, the extraction of the encoded signals by the example receiver circuit 26 is performed using a frequency separator circuit, and also decision circuitry. The frequency separator circuit may be provided by a demodulator, such as an FM demodulator, for example, and separates the different carrier frequencies of the different transmitter circuits. The extracted signal generated by the sensor in response to the detection of the arcing event by the sensor, and the extracted identity of the sensor, are then outputted by the decision circuit and communicated to the processing unit 14.

In this example, the sensors and their corresponding transmitter circuits 24 are disposed in known locations with respect to the medical imaging system. Consequently, the processing unit 14, can determine the location of the arcing event within the medical imaging system based on the identities of the sensors from which the signals are received. As mentioned above, the encoded signals may represent the signal itself that is generated by the sensor in response to a detection of an arcing event by the sensor, or they may represent the detected energy of the signal generated by the sensor, or they may represent the amplitude of the signal generated by the sensor. These encoded signals are used by the processing unit 14 to determine the location of the arcing event within the medical imaging system. A sensor that is relatively closer to the origin of the arcing event is expected to generate a signal with relatively higher amplitude, and also energy, than a sensor that is relatively further from the origin of the arcing event. Thus, the processing unit 14 may for instance assign the location of the arcing event to the sensor that generates a signal that has the largest amplitude, or energy. This may be assessed by the processing unit 14 based on the extracted signal itself, or based on the extracted energy, or the extracted amplitude.

In one example, the encoded signals represent i) a time of generation of the signal by the sensor in response to the detection of the arcing event by the sensor and/ or an energy, or an amplitude, of the signal generated by the sensor in response to the detection of the arcing event by the sensor. In this example, the at least one processing unit 14 is configured to determine the location of the arcing event within the medical imaging system based on: the difference between signals received from a plurality of the sensors. In this example, the difference is calculated based on the difference between a time of generation of the signal by the sensors in response to the detection of the arcing event by the sensors and/ or the difference between an energy, or an amplitude, of the signal generated by the sensors in response to the detection of the arcing event by the sensors.

In this example, the difference between a time of generation of the signal by the sensors in response to the detection of the arcing event by the sensors facilitates a triangulation to be performed on the location of the arcing event. The difference between the energy, or the amplitude, of the signal generated by the sensors in response to the detection of the arcing event by the sensors, facilitates a comparison between the signals, the result of which can be to assign the location of the arcing event to the sensor that has detected the signal with the highest amplitude, or energy.

The algorithms as described herein may be executed locally on dedicated hardware platforms (e.g., edge devices) or executed in the cloud (e.g., by sending the raw sensor signals to the cloud). Any intermediate distribution of algorithm elements between cloud and on-premise is also foreseen.

The defect localisation system as described herein may have different operation modes, which may include continuous monitoring during diagnostic scanning, monitoring at dedicated well defined and repeatable test-sequences outside of the diagnostic scans, and special service operation modes.

For calibration of the defect localization system, various imaging and non-imaging linear or helical scans can be used, e.g. tube conditionings (no gantry rotation), scout scans, air calibration scans and some of the most common imaging scans.

The defect localisation system as described herein may be suitable to remotely detect events and defects and to support root-cause identification. It may allow monitoring of systems and act pro-actively, even before the problem occurs to the users (e.g., clinical users) of the system. For example, in the case of soft-arcing it may be possible to prevent further deterioration or reoccurrence by so-called tube conditioning, or by changing settings (e.g., changing high voltage settings) in the system. In the case remote resolution of the problem is not possible anymore, the number of FSE visits can be reduced (ideally to only 1 visit) and the amount of to-be-shipped spare-parts can be minimized. In addition, the logistic supply chain of spare parts can be optimized by timely shipment of parts to the right continent or country.

Fig. 11 illustrates a flowchart describing an exemplary computer-implemented method 200 of determining a location of a potential failure event within a medical imaging system. The computer-implemented method as described herein is also applicable for various types of failure event detection. For example, the computer-implemented method is applicable for arc localisation. For example, the computer-implemented method as described herein may be applicable for detecting and localising a worn component that causes noise and vibration. For examples, the computer-implemented method as described herein may be applicable for detecting and localising a mechanical component operating with changing noise signals or audio signals due to one or more of changing environmental conditions, changing temperature condition, changing subcomponent aging condition, changing location, and/or grid voltage variation. For examples, the computer-implemented method as described herein may be applicable for detecting and localising a cooling fluid problem that generates characteristic noises upon gantry rotation. For example, the computer-implemented method as described herein may be applicable for detecting and localising a contamination of a cooling fluid that causes a characteristic sound. For example, the computer-implemented method as described herein may be applicable for detecting and localising a thermal problem due to clogged filters in air circulation component.

At block 210, at least one processing unit (e.g., the at least processing unit 14 shown in Fig. 3) receives a signal generated by the one or more sensors. The one or more sensors comprise one or more acoustic sensors, one or more a vibration sensors, one or more RF sensors, or any combination thereof.

In some examples, the one or more sensors are mountable at one or more locations on a stationary part of the medical imaging system. In some examples, the one or more sensors are mountable at one or more locations on at one or more external locations in a room, in which the medical imaging system is disposed.

At block 220, the at least one processing unit determines a location of a potential failure event within the medical imaging system based on a signature in the signal received from the one or more sensors. For example, it is possible to classify key features in the waveform of the defect signal itself, which are characteristic for the signal source. In this way, characteristic fingerprints of e.g. arcing in X-ray tubes and power supplies, may be identified and used for remote differential diagnostics and first-time right replacement of failed parts.

Alternatively or additionally, the at least one processing unit determines a location of a potential failure event within the medical imaging system based on a difference between the signals received from a plurality of the sensors. In some examples, the difference between the signals received from the plurality of sensors may comprise a difference in amplitude between the signals received from the plurality of sensors. In some examples, the difference between the signals received from the plurality of sensors may comprise a difference in phase between the signals received from the plurality of sensors. In some examples, the difference between the signals received from the plurality of sensors may comprise a difference in time of arrival between the signals received from the plurality of sensors.

In some examples, the medical imaging system may comprise a movable part. The potential failure event comprises a potential failure event on the movable part within the medical imaging system. The at least one processing unit is configured to receive a signal indicative of position information of the movable part within the medical imaging system and to determine a location of the potential failure event on the movable part within the medical imaging system based on the position information. For example, the medical imaging system comprises a CT imaging system and the movable part within the medical imaging system comprises a rotatable gantry of the CT imaging system. For example, the medical imaging system is an X-ray imaging system, and the movable part within the medical imaging system comprises a movable support of the X-ray imaging system.

The position of the moving object can be derived from one or more of the following exemplary signals.

In some examples, the signal indicative of position information of the movable part may comprise a reading from a gantry or movable support position encoder.

In some examples, the signal indicative of position information of the movable part may comprise an audio, vibrational, and/or radio-frequency signal that is produced by a component of the movable part of the medical imaging system. The at least one processing unit may be configured to identify the location of the potential failure event based on a known position of the component of the movable part within the medical imaging system with respect to the one or more sensors.

In some examples, at least one beacon is attachable to the movable part. The signal indicative of position information of the movable part comprises an audio signal, a vibrational signal, and/or a radio-frequency signal generated by the at least one attached beacon. The at least one beacon may be provided with an acceleration sensor usable to provide sensor data indicative of a movable part spatial orientation.

The at least one processing unit may be configured to determine the location of the potential failure event within the medical imaging system based further on a model representing a relative location of the one or more sensors with respect to one or more components of the medical imaging system and/or one or more beacons.

A communication channel may be provided to connect the at least one processing unit to a remote service application. The at least one processing unit is configured to provide information about the detected potential failure event via the communication channel to the remote service application for generating a service work order and/or for training a predictive model for detecting a potential future system issue. The service work order may comprise one or more of automatic service scheduling, part ordering, and proposal of a change in a hospital's workflow.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A defect localisation system (10) for determining a location of a potential failure event within a medical imaging system, the defect localisation system comprising:
- one or more sensors (12); and
- at least one processing unit (14);
wherein the one or more sensors comprise at least one of an acoustic sensor (12a), a vibration sensor (12b), and a radio-frequency sensor; and
wherein the at least one processing unit is configured to receive a signal generated by the one or more sensors, and to determine a location of a potential failure event within the medical imaging system based on:
a) a signature in the signal received from the one or more sensors; and/or
b) a difference between signals received from a plurality of the sensors.

2. The defect localisation system according to claim 1,
wherein the one or more sensors are mountable at one or more locations on a stationary part of the medical imaging system and/or at one or more external locations in a room, in which the medical imaging system is disposed.

3. The defect localisation system according to claim 1 or 2,
wherein the medical imaging system comprises a movable part;
wherein the potential failure event comprises a potential failure event on the movable part within the medical imaging system; and
wherein the at least one processing unit is configured to receive a signal indicative of position information of the movable part within the medical imaging system and to determine a location of the potential failure event on the movable part within the medical imaging system based on the position information.

4. The defect localisation system according to claim 3,
wherein the medical imaging system comprises a computed tomography, CT imaging system or an X-ray imaging system, and the movable part within the medical imaging system comprises a rotatable gantry of the CT imaging system or a movable support of the X-ray imaging system.

5. The defect localisation system according to claim 4,
wherein the signal indicative of position information of the movable part comprises a reading from a gantry or movable support position encoder.

6. The defect localisation system according to any one of claims 3 to 5, further comprising:
- at least one beacon (114) that is attachable to the movable part, wherein the signal indicative of position information of the movable part comprises an audio signal, a vibrational signal, and/or a radio-frequency signal generated by the at least one attached beacon.

7. The defect localisation system according to claim 6,
wherein the at least one beacon is provided with an acceleration sensor usable to provide sensor data indicative of a movable part spatial orientation.

8. The defect localisation system according to any one of claims 3 to 7,
wherein the signal indicative of position information of the movable part comprises an audio, vibrational, and/or radio-frequency signal that is produced by a component of the movable part of the medical imaging system; and
wherein the at least one processing unit is configured to identify the location of the potential failure event based on a known position of the component of the movable part within the medical imaging system with respect to the one or more sensors.

9. The defect localisation system according to claim 1 or claim 2,
wherein the potential failure event is an arcing event;
wherein the defect localisation system comprises a plurality of the sensors (12), and each sensor further comprises a transmitter circuit, the transmitter circuit being configured to transmit encoded signals for the sensor, the encoded signals representing i) the signal generated by the sensor in response to a detection of the arcing event by the sensor, and ii) an identity of the sensor; and
wherein the defect localisation system further comprises a receiver circuit, the receiver circuit being configured to:
receive the encoded signals transmitted by the transmitter circuits;
extract from the encoded signals: i) the signal generated by the sensor in response to the detection of the arcing event by the sensor, and ii) the identity of the sensor; and
communicate to the at least one processing unit (14): i) the extracted signal generated by the sensor in response to the detection of the arcing event by the sensor, and ii) the extracted identity of the sensor; and
wherein the at least one processing unit (14) is further configured to receive the identity of the sensor for the signals generated by the sensors, and to determine the location of the arcing event within the medical imaging system based further on c) the identities of the sensors from which the signals are received.

10. The defect localisation system according to claim 9, wherein the encoded signals represent i) a time of generation of the signal by the sensor in response to the detection of the arcing event by the sensor and/ or an energy, or an amplitude, of the signal generated by the sensor in response to the detection of the arcing event by the sensor; and
wherein the at least one processing unit (14) is configured to determine the location of the arcing event within the medical imaging system based on:
b) the difference between signals received from a plurality of the sensors, the difference being calculated based on the difference between a time of generation of the signal by the sensors in response to the detection of the arcing event by the sensors and/ or the difference between an energy, or an amplitude, of the signal generated by the sensors in response to the detection of the arcing event by the sensors.

11. The defect localisation system according to any one of claims 1 - 8,
wherein the potential failure event comprises one or more of:
- an arcing event;
- a worn component that causes noise and vibration;
- a mechanical component operating with changing noise signals or audio signals;
- a cooling fluid problem that generates characteristic noises upon gantry rotation;
- a contamination of a cooling fluid that causes a characteristic sound; and
- a thermal problem due to clogged filters in air circulation component.

12. The medical imaging system according to any one of the preceding claims,
wherein the difference between the signals received from the plurality of sensors comprises one or more of:
- a difference in amplitude between the signals received from the plurality of sensors;
- a difference in phase between the signals received from the plurality of sensors; and
- a difference in time of arrival between the signals received from the plurality of sensors.

13. The defect localisation system according to any one of the preceding claims, further comprising:
- a communication channel (16) configured to connect the at least one processing unit to a remote service application; and
wherein the at least one processing unit is configured to provide information about the detected potential failure event via the communication channel to the remote service application for generating a service work order and/or for training a predictive model for detecting a potential future system issue.

14. The defect localisation system according to claim 13,
wherein the service work order comprises one or more of:
- automatic service scheduling;
- part ordering; and
- proposal of a change in a hospital's workflow.

15. The defect localisation system according to any one of claims 1 to 14,
wherein the at least one processing unit is configured to determine the location of the potential failure event within the medical imaging system based further on a model representing a relative location of the one or more sensors with respect to one or more components of the medical imaging system and/or one or more beacons.

16. A medical imaging arrangement, comprising:
- a medical imaging system (100); and
- a defect localisation system (10) according to any one of the preceding claims.

17. A computer-implemented method (200) of determining a location of a potential failure event within a medical imaging system, the method comprising:
- receiving (210), by at least one processing unit, a signal generated by the one or more sensors, wherein the one or more sensors comprise at least one of an acoustic sensor, a vibration sensor, or a radio-frequency sensor; and
- determining (220), by the at least one processing unit, a location of a potential failure event within the medical imaging system based on:
a) a signature in the signal received from the one or more sensors; and/or
b) a difference between the signals received from a plurality of the sensors.
